# EUROPEAN PATENT SPECIFICATION

(11) **EP 1 131 055 B1**
(45) Date of publication and mention of the grant of the patent: **09.11.2005**
(21) Application number: 99971701.0
(22) Date of filing: 09.11.1999
(51) Int. Cl.: A61K 9/14, A61K 38/17

(54) **TREATMENT SET CONTAINING LUNGSURFACTANT COMPOSITIONS**
LUNGENSURFACTANT ZUBEREITUNGEN ENTHALTENDES BEHANDLUNGSET
APPAREIL DE TRAITEMENT CONTENANT DES COMPOSITIONS A BASE DE SURFACTANTS PULMONAIRES

(30) Priority: 10.11.1998 EP 98121324
(43) Date of publication of application: 12.09.2001
(73) Proprietor: ALTANA Pharma AG, 78467 Konstanz (DE)
(72) Inventor: GERMANN, Paul-Georg, D-78549 Lörrach (DE); RUPP, Herbert, D-78465 Konstanz (DE); EISTETTER, Klaus, D-78465 Konstanz (DE); KILIAN, Ulrich, D-78479 Reichenau (DE); HÄFNER, Dietrich, D-78464 Konstanz (DE)
(86) International application number: PCT/EP1999/008567
(87) International publication number: WO 2000/027360

(56) References cited:
- WO-A-97/26863
- GOMMERS D, EIJKING EP, SO KL, VAN'T VEEN A, LACHMANN B: "Bronchoalveolar lavage with a diluted surfactant suspension prior to surfactant instillation improves the effectiveness of surfactant therapy in experimental acute respiratory distress syndrome (ARDS)" INTENSIVE CARE MEDICINE, vol. 24, no. 5, May 1998 (1998-05), pages 494-500, XP002099318 cited in the application

## Description

### Technical field of the invention

The invention relates to a set for the treatment of disease conditions, which are described as Infant Respiratory Distress Syndrome (IRDS) and Acute or Adult Respiratory Distress Syndrome (ARDS), with pulmonary surfactant preparations.

### Prior art

ARDS (Adult Respiratory Distress Syndrome) is a descriptive expression which is applied to a large number of acute, diffusively infiltrative pulmonary lesions of differing etiology if they are connected with a severe gas exchange disorder (in particular arterial hypoxemia). The expression ARDS is used because of the numerous clinical and pathological factors common with IRDS (Infant Respiratory Distress Syndrome). If, in IRDS, the pulmonary surfactant deficiency caused by premature birth is predominant, in ARDS a pulmonary surfactant malfunction is caused by the disorder of the lung, which is based on differing etiologies.

For many years, it has proven suitable to treat IRDS by introducing pulmonary surfactant preparations into the lungs of the affected children. It is known from pilot studies that pulmonary surfactant preparations are also clinically effective in ALI (Acute Lung Injury) including ARDS (review, for example, B. Lachmann, D. Gommers and E.P. Eijking: Exogenous surfactant therapy in adults, Atemw.-Lungenkrkh. 1993, 19:581-91; D. Walmrath et al.: Bronchoscopic surfactant administration in patients with severe adult respiratory distress syndrome and sepsis, Am. J. Respir. Crit. Care Med. 1996, 154:57-62; T.J. Gregory et al.: Bovine surfactant therapy for patients with acute respiratory distress syndrome, Am. J. Respir. Crit. Care Med. 1997, 155:1309-15). In this type of treatment, the pulmonary surfactant is either instilled intratracheally as a bolus (IRDS and ARDS) or instilled into individual sections of lung via a bronchoscope (ARDS).

V. Balaraman et al. (Physiologic response and lung distribution of lavage versus bolus Exosurf® in piglets with acute lung injury, Am. J. Respir. Crit. Care Med 1996, 153:1838-43) describe the administration of pulmonary surfactant in an animal model by rinsing in and subsequent drainage. Gommers et al. [Bronchoalveolar lavage with a diluted surfactant suspension prior to surfactant instillation improves the effectiveness of surfactant therapy in experimental acute respiratory distress syndrome (ARDS), Intensive Care Med. 1998, 24:494-500] describe the carrying-out of a bronchoalveolar lavage (BAL) with dilute surfactant suspension from natural surfactant. Natural pulmonary surfactant is then instilled.

### Description of the invention

The object of the invention is the provision of a set for the treatment of IRDS and ARDS by bronchoalveolar lavage with dilute pulmonary surfactant solution and subsequent administration of pulmonary surfactant.

It has been found that this object is achieved by a set comprising a first container which has a volume of 50 to 500 ml and contains a pulverulent pulmonary surfactant preparation, the amount of phospholipids in the surfactant being 50 to 500 mg, and a second container which has a volume of 50 to 500 ml and contains a pulverulent pulmonary surfactant preparation, the amount of phospholipids in the surfactant of the second container being 1 to 10 g and wherein a pulmonary surfactant protein is only contained in the second container.

Further subjects of the invention are contained in the subclaims.

According to the invention, the terms ALI, ARDS and IRDS are understood as meaning the abovementioned disease conditions, where the term ALI is also intended to include ARDS.

Natural pulmonary surfactant has surface-active properties; it reduces, for example, the surface tension in the pulmonary alveoli. The simple and rapid in vitro test with which the surface activity of pulmonary surfactant can be determined is, for example, the so-called Wilhelmy balance [J. Goerke, Biochim. Biophys. Acta, 344:241-261 (1974), R.J. King and J.A. Clements, Am. J. Physicol. 223: 715-726 (1972)]. This method gives information on the pulmonary surfactant quality, measured as the ability of a pulmonary surfactant to reach a surface tension of nearly zero mN/m. Another measuring device to determine the surface activity of pulmonary surfactant is the "Pulsating Bubble Surfactometer" [F. Possmayer, S. Yu and M. Weber, Prog. Resp. Res., Ed. v. Wichert, Vol. 18: 112-120 (1984)].

The activity of a pulmonary surfactant composition can also be determined by means of in vivo tests, for example as described in the following section "Pharmacology". By the measurement of, for example, the pulmonary compliance, the blood gas exchange or the respiratory pressures needed, it is possible to obtain information on the activity of a pulmonary surfactant.

Pulmonary surfactant preparations are understood according to the invention as meaning the numerous known compositions and their modificatibns which have the function of natural pulmonary surfactant. Preferred compositions here are those which, for example, have activity in the tests described above. Particularly preferred compositions are those which exhibit an increased activity in such a test in comparison with natural, in particular human, pulmonary surfactant. In this connection, they can be compositions that only contain phospholipids, but also compositions which, apart from the phospholipids, inter alia, additionally contain pulmonary surfactant protein. Commercial products which may be mentioned are Curosurf® (Serono, Pharma GmbH, Unterschleißheim), a highly purified natural surfactant from homogenized pigs' lungs, Survanta® (Abbott GmbH, Wiesbaden) and Alveofact® (Dr. Karl Thomae GmbH Biberach), both extracts of bovine lungs, and Exosurf® (Deutsche Wellcome GmbH, Burgwedel), a synthetic phospholipid with excipients. Possible pulmonary surfactant proteins are both those obtained from natural sources, such as, for example, pulmonary lavage or extraction from amniotic fluid, and the proteins prepared by genetic engineering or chemical synthesis. According to the invention, the pulmonary surfactant proteins designated by SP-B and SP-C and their modified derivatives are particularly of interest. The amino acid sequences of these pulmonary surfactant proteins and their isolation or production by genetic engineering are known (e.g. from WO86/03408, EP-A-0 251 449, WO89/04326, WO87/06943, WO88/03170, WO91/00871, EP-A-0 368 823 and EP-A-0 348 967). Modified derivatives of the pulmonary surfactant proteins designated by SP-C, which differ from human SP-C by the replacement of a few amino acids, are described, for example, in WO91/18015 and WO95/32992. Particularly to be emphasized in this connection are the recombinant SP-C derivatives which are disclosed in WO95/32992, in particular those which differ from human SP-C in positions 4 and 5 by the replacement of cysteine by phenylalanine and in position 32 by the replacement of methionine by isoleucine [in the following designated as rSP-C (FF/I). Modified derivatives of the pulmonary surfactant proteins are also to be understood as meaning those proteins which have an amino acid sequence which is conceived completely independently with respect to its pulmonary surfactant property, such as are described, for example, in EP-A-0 593 094 and WO 92/22315. In this connection, the polypeptide KL4 (INN: sinapultide) may be particularly mentioned. EP-B-0 100 910, EP-A-0 110 498, EP-B-0 119 056, EP-B-0 145 005 and EP-B-0 286 011 describe phospholipid compositions with and without pulmonary surfactant proteins, which are also suitable as components of the preparations.

According to the invention, the pulverulent pulmonary surfactant preparations are lyophilized and, in particular, spray-dried pulmonary surfactant preparations. Lyophilized preparations are disclosed, for example, in WO 97/35882, WO 91/00871 and DE 3229179. WO 97/26863 describes a process for the preparation of pulverulent pulmonary surfactant preparations by spray-drying. According to the invention, preparations prepared in this way are preferred. Preferred preparations according to the invention contain 80 to 95 % by weight of phospholipids, 0.5 to 3.0 % by weight of pulmonary surfactant proteins, 3 to 15 % by weight of fatty acid, preferably palmitic acid, and 0 to 3 % by weight of calicum chloride.

The pulmonary surfactant preparations in the first and in the second container can be pulmonary surfactant preparations of identical or different composition. In this connection, a treatment set may be preferably mentioned which in the first container (for the rinsing solution) contains a pulmonary surfactant preparation which has no pulmonary surfactant proteins (e.g. a composition such as is on the market under the name Exosurf®) and in the second container contains a pulmonary surfactant preparation which has one or more pulmonary surfactant proteins, preferably rSP-C (FF/I). The expensive pulmonary surfactant proteins in the preparation can thus be saved.

According to the invention, the container is preferably a transparent container made of a suitable material, which makes possible observation of the resuspension of the pulmonary surfactant preparation on addition of solvent. Suitable materials which may be mentioned are transparent polyethylene and preferably glass. Particularly preferably, the container is a glass bottle, which can be sealed, for example, by a customary rubber stopper or a septum. The first and the second containers can have an identical or different volume. A scale and/or a suitable imprint (e.g. rinsing solution or instillation solution) can also be applied to the container.

The first container serves for the preparation of the solution for the bronchoalveolar lavage (rinsing). For this purpose, the pulverulent pulmonary surfactant preparation is resuspended by addition of a suitable solvent to the container. Preferably, the suitable solvent is a physiological saline solution, of which, depending on the container size and the amount of pulmonary surfactant contained, between 50 and 500 ml, preferably 50 to 250 ml, are added. A suitable amount for the lavage can be taken from the container and the lungs rinsed therewith. The rinsing can be carried out in a known manner, for example, using a bronchoscope. It has proven advantageous for the bronchoalveolar lavage to use 50 to 100 ml of a solution which contains 1 to 5 mg of a phospholipid per ml of solution.

The second container serves for the preparation of the solution for the administration of the pulmonary surfactant preparation. For this purpose, the pulverulent pulmonary surfactant preparation is resuspended by addition of a suitable solvent to the container. Preferably, the suitable solvent is a physiological saline solution, of which, depending on the container size and the amount of pulmonary surfactant contained, between 50 and 500 ml, preferably 50 to 250 ml, are added. A suitable amount for administration can be taken from the container. The administration is preferably carried out by intratracheal instillation, infusion, bolus or in the form of an atomization. It has proven advantageous to administer 50 to 100 ml of a solution which contains 25 to 100 mg of phospholipid per ml of solution.

Adult Respiratory Distress Syndrome (ARDS) is a descriptive expression which is applied to a large number of acute, diffuse infiltrative pulmonary lesions of differing etiology if they are associated with a severe gas exchange disorder (in particular arterial hypoxemia). The expression ARDS is used because of the numerous clinical and pathological features common with Infant Respiratory Distress Syndrome (IRDS). If, in the case of IRDS, the lung surfactant deficiency caused by premature birth is predominant, then in the case of ARDS a lung surfactant malfunction is caused by the lung condition based on differing etiologies.

Triggering causes for ALI (Acute Lung Injury) including ARDS can, for example, be (cited in accordance with Harrison's Principles of Internal Medicine 10th Ed. 1983 McGraw-Hill Int. Book Comp.) diffuse pulmonary infections (e.g. due to viruses, bacteria, fungi), aspiration of, for example, gastric juice or in the case of near-drowning, inhalation of toxins or irritants (e.g. chlorine gas, nitrogen oxides, smoke), direct or indirect trauma (e.g. multiple fractures or pulmonary contusion), systemic reactions to inflammations outside the lung (e.g. hemorrhagic pancreatitis, gram-negative septicemia), transfusions of high blood volumes or alternatively after cardiopulmonary bypass.

The sets for treatment according to the invention are not only suitable for the treatment or prophylaxis of IRDS in premature babies and for the treatment or prophylaxis of ALI including ARDS in adults, but also for the treatment or prophylaxis of pneumonia, bronchitis, meconium aspiration syndrome, COPD (chronic obstructive pulmonary disease), asthma and cystic fibrosis.

The invention also therefore further relates to a method for the treatment or prophylaxis of ALI including ARDS in adults, the treatment or prophylaxis of IRDS in premature babies, the treatment or prophylaxis of pneumonia, bronchitis, meconium aspiration syndrome, COPD, asthma and cystic fibrosis by bronchoalveolar lavage with dilute pulmonary surfactant solution and subsequent administration of pulmonary surfactant. Preferably, this treatment is carried out twice daily over a period of 1 to at most 5 days. A method is preferred in which the dilute pulmonary surfactant solution employed contains 1 to 10 mg of phospholipids per ml of solvent and the subsequently administered pulmonary surfactant contains 25 to 100 mg of phospholipids per ml of solvent. A method is particularly preferred in which the dilute pulmonary surfactant solution employed contains no or additionally 0.02 to 1.0 mg of rSP-C (FF/I) per ml of solvent and the subsequently administered pulmonary surfactant additionally contains 0.5 to 2.0 mg of rSP-C (FF/I) per ml of solvent. A method may be particularly mentioned in which the dilute pulmonary surfactant solution employed contains no or 0.05 mg of rSP-C (FF/I) per ml of solvent and the subsequently administered pulmonary surfactant contains 1 mg of rSP-C (FF/I) per ml of solvent.

Further subject of the invention is an article of manufacture comprising customary secondary packaging material and a set for treatment according to the invention contained within the secondary packaging material, optionally together with suitable pharmaceutical auxiliaries (for example saline solution for resuspension of active agents in case of powdered forms), and optionally together with a suitable device for administration of the pulmonary surfactant solution and wherein a container of the set for teatment and/or the secondary packaging material comprises a label or package insert which indicates that the pharmaceutical preparation contained in the container is useful for preventing or treating pneumonia, bronchitis, meconium aspiration syndrome, COPD (chronic obstructive pulmonary disease), asthma, cystic fibrosis, IRDS and/or ALI (including ARDS). The secondary packaging material, the containers and the label or package insert may comply with what is considered as standard for pharmaceutical compositions of this kind by those skilled in the art.

### Examples

### A.) Production of pulverulent pulmonary surfactant preparations

Pulverulent pulmonary surfactant preparations are produced according to the process described in WO 97/26863:

### Example 1

7.0 g of 1,2-dipalmitoyl-3-sn-phosphatidylcholine, 2.5 g of 1-palmitoyl-2-oleoyl-3-sn-phosphatidylglycerol-sodium, 205 mg of calcium chloride dihydrate and 250 mg of palmitic acid are dissolved in 300 ml of ethanol/water (85:15) with warming to 60°C, and the solution is cooled to room temperature and mixed with 350 ml of a solution of rSP-C (FF/I) in chloroform/methanol 9:1 (c = 429 mg/l). The resulting solution is spray-dried in a Büchi B 191 laboratory spray dryer. Spray conditions: drying gas air, inlet temperature 90°C, outlet temperature 52-54°C. A loose powder is obtained.

### Example 2

A solution of surfactant obtained from bovine lungs (obtained by extraction and purification steps, such as described, for example, in EP 406732) in chloroform/methanol is spray-dried under the following conditions: Büchi B 191, laboratory spray dryer, drying gas nitrogen, inlet temperature 80°C, outlet temperature 50-52°C. A fine, yellowish powder is obtained.

### Example 3

10.95 g of 1,2-dipalmitoyl-3-sn-phosphatidylcholine, 4.6 g of 1-palmitoyl-2-oleoyl-3-sn-phosphatidylglycerol-ammonium, 418 mg of calcium chloride dihydrate and 750 mg of palmitic acid are dissolved at 50°C in 330 ml of 2-propanol/water (85:15) and, after cooling to 30°C, the solution is mixed with 620 ml of a solution of rSP-C (FF/I) in isopropanol/water (95:5, c= 484 mg/l). The resulting solution is spray-dried in a Büchi B 191 laboratory spray dryer. Spray conditions: drying gas nitrogen, inlet temperature 100°C, outlet temperature 58-60°C. A colorless powder is obtained.

### Example 4

3.74 g (5.1 mmol) of 1,2-dipalmitoyl-3-sn-phosphatidylcholine, 2.81 g (3.7 mmol) of 1-palmitoyl-2-oleo-yl-3-sn-phosphatidylcholine, 2.90 g (3.9 mmol) of 1,2-dipalmitoylphosphatidyl-3-sn-phosphatidylglycerol-sodium, 234 mg of palmitic acid and 279 mg (1.9 mmol) of calcium chloride dihydrate are dissolved at 50°C in 160 ml of 2-propanol/water (85:15) and, after cooling to 30°C, the solution is mixed at 30°C with 566 ml of a solution of rSP-C (FF/I) in isopropanol/water (92:8, c= 330 mg/I). The resulting solution is spray-dried in a Büchi B 191 laboratory spray dryer. Spray conditions: drying gas nitrogen, inlet temperature 90°C, outlet temperature 58-60°C. A colorless powder is obtained.

### Example 5

0.5 g of RLLLLRLLLLRLLLLRLLLLR (R = Arg, L = Leu) or KLLLLKLLLLKLLLLKLLLLK (K = lysine; L = leucine), 7.125 g of 1,2-dipalmitoyl-3-sn-phosphatidylcholine and 2.43 g of 1-palmitoyl-2-oleoyl-3-sn-phosphatidylglycerol-ammonium are dissolved in 500 ml of chloroform/methanol 1:1 with warming to 45°C and then spray-dried in a Büchi B 191 laboratory spray dryer. Spray conditions: drying gas nitrogen, inlet temperature 85°C, outlet temperature 55°C. A colorless powder is obtained.

### Example 6

A solution of phospolipids, palmitic acid and calcium chloride dihydrate obtainable according to Example 1, 3 or 4 is spray-dried - without addition of a solution of rSP-C (FF/I) - according to the conditions in Example 1, 3 or 4. A powder is obtained.

### B.) Preparation of the treatment sets:

50 to 500 mg of the powder obtained in Example 1 are filled into a first bottle having a volume of 250 ml and the imprint rinsing solution and the bottle is sealed. 1 to 10 g of the powder obtained in Example 1 are filled into a second bottle having a volume of 250 ml and the imprint instillation solution and the bottle is sealed.

100 to 1000 mg of the powder obtained in Example 2 are filled into a first bottle having a volume of 500 ml and the imprint rinsing solution and the bottle is sealed. 2 to 20 g of the powder obtained in Example 2 are filled into a second bottle having a volume of 250 ml and the stamp instillation solution and the bottle is sealed.

50 to 500 mg of the powder obtained in Example 3 are filled into a first bottle having a volume of 100 ml and the stamp rinsing solution and the bottle is sealed. 1 to 10 g of the powder obtained in Example 3 are filled into a second bottle having a volume of 100 ml and the stamp instillation solution and the bottle is sealed.

50 to 500 mg of the powder obtained in Example 4 are filled into a first bottle having a volume of 250 ml and the stamp rinsing solution and the bottle is sealed. 1 to 10 g of the powder obtained in Example 4 are filled into a second bottle having a volume of 250 ml and the stamp instillation solution and the bottle is sealed.

50 to 100 mg of the powder obtained in Example 5 are filled into a first bottle having a volume of 250 ml and the stamp rinsing solution and the bottle is sealed. 2 to 20 g of the powder obtained in Example 5 are filled into a second bottle having a volume of 250 ml and the stamp instillation solution and the bottle is sealed.

50 to 500 mg of the powder obtained in Example 1 are filled into a first bottle having a volume of 250 ml and the stamp rinsing solution and the bottle is sealed. 1 to 10 g of the powder obtained in Example 3 are filled into a second bottle having a volume of 250 ml and the stamp instillation solution and the bottle is sealed.

100 to 1000 mg of the powder obtained in Example 2 are filled into a first bottle having a volume of 500 ml and the stamp rinsing solution and the bottle is sealed. 2 to 20 g of the powder obtained in Example 1 are filled into a second bottle having a volume of 250 ml and the stamp instillation solution and the bottle is sealed.

50 to 500 mg of the powder obtained in Example 3 are filled into a first bottle having a volume of 100 ml and the stamp rinsing solution and the bottle is sealed. 1 to 10 g of the powder obtained in Example 5 are filled into a second bottle having a volume of 100 ml and the stamp instillation solution and the bottle is sealed.

50 to 500 mg of the powder obtained in Example 4 are filled into a first bottle having a volume of 250 ml and the stamp rinsing solution and the bottle is sealed. 1 to 10 g of the powder obtained in Example 1 are filled into a second bottle having a volume of 250 ml and the stamp instillation solution and the bottle is sealed.

50 to 100 mg of the powder obtained in Example 5 are filled into a first bottle having a volume of 250 ml and the stamp rinsing solution and the bottle is sealed. 2 to 20 g of the powder obtained in Example 1 are filled into a second bottle having a volume of 250 ml and the stamp instillation solution and the bottle is sealed.

50 to 500 mg of a powder obtained in Example 6 are filled into a first bottle having a volume of 250 ml and the stamp rinsing solution and the bottle is sealed. 1 to 10 g of the powder obtained in Example 1 are filled into a second bottle having a volume of 250 ml and the stamp instillation solution and the bottle is sealed.

100 to 1000 mg of a powder obtained in Example 6 are filled into a first bottle having a volume of 500 ml and the stamp rinsing solution and the bottle is sealed. 2 to 20 g of the powder obtained in Example 2 are filled into a second bottle having a volume of 250 ml and the stamp instillation solution and the bottle is sealed.

50 to 500 mg of a powder obtained in Example 6 are filled into a first bottle having a volume of 100 ml and the stamp rinsing solution and the bottle is sealed. 1 to 10 g of the powder obtained in Example 3 are filled into a second bottle having a volume of 100 ml and the stamp instillation solution and the bottle is sealed.

50 to 100 mg of powder obtained in Example 6 are filled into a first bottle having a volume of 250 ml and the stamp rinsing solution and the bottle is sealed. 2 to 20 g of the powder obtained in Example 5 are filled into a second bottle having a volume of 250 ml and the stamp instillation solution and the bottle is sealed.

### Pharmacology

In IRDS, ALI and ARDS, the formation of hyaline membranes and a heavy infiltration of inflammatory cells is observed. It was found that lavage with dilute surfactant solution brings about a cleansing of the lung of those proteins and inflammatory cells which are observed in the lung of patients having the abovementioned syndromes. The subsequently administered pulmonary surfactant then meets an almost pathologically perfect lung and exhibits a more pronounced action than on administration without prior rinsing.

In an animal model of ARDS [D. Häfner et al., Am J Resp Crit Care Med 158:270-278, (1998)], in which histological investigations were carried out, a strong formation of hyaline membranes, a strong formation of edemas and a severe infiltration of inflammatory cells into the lungs of lavaged animals (both changes such as can be observed in ARDS in man) are seen. These histopathological changes lead to the impairment of gas exchange, which is detectable as a decreased arterial oxygen partial pressure and increased arterial carbon dioxide partial pressure in the blood. Based on these changes, approaches were sought which could eliminate these phenomena. Starting from a possible cleansing of the lung with surfactant rinsing solution, the idea was developed that the combination of a rinsing solution and subsequent instillation of, for example, rSP-C surfactant could act additively in the syndromes IRDS and ARDS.

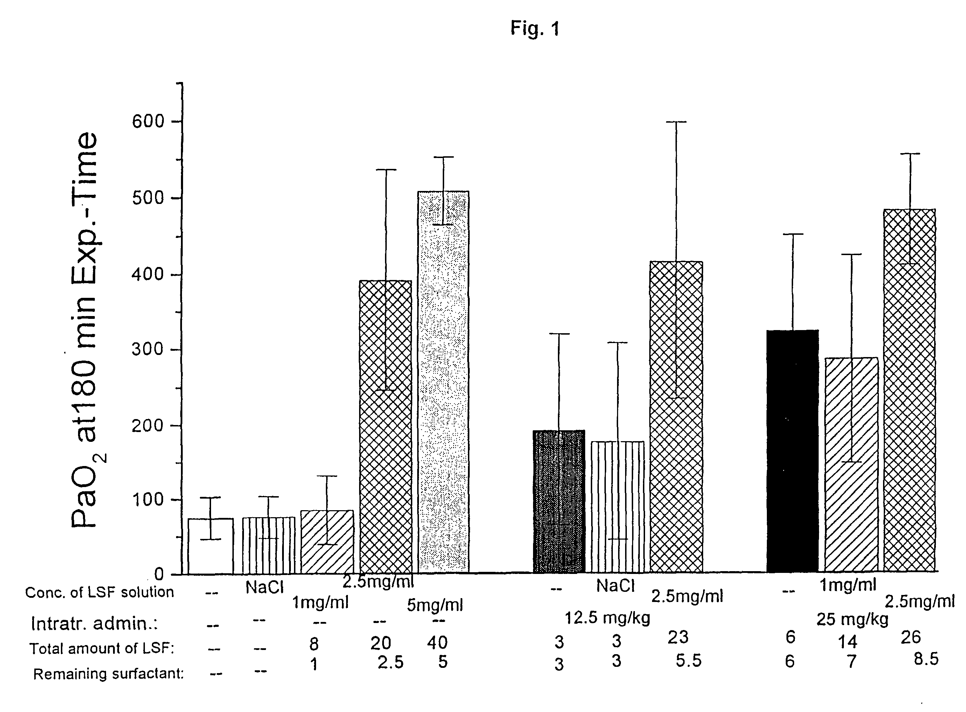

In this animal model of ARDS (repeated pulmonary lavage in rats), it was possible to demonstrate the abovementioned pathological changes by means of histological investigations. These histological changes were accompanied by changes in gas exchange (reduced arterial oxygen partial pressure with increased arterial carbon dioxide partial pressure). Various concentrations of rinsing solution were used leading to the results shown in Figure 1. 60 minutes after the last lavage with which the acute lung damage was induced, the lungs of the animals were treated with the respective rinsing solutions. For this purpose, 8 ml of solution in each case (with the concentrations indicated) were used in animals having a body weight of about 250 g. A group that received lavage with NaCl solution only served as additional control group. Of the 8 ml instilled, about 7 ml in each case were recovered again. Thus about 1 ml of rinsing solution always remained in the lung. Figure 1 illustrates that a rinsing solution with rSP-C (FF/I) surfactant in a concentration of 100 µg of rSP-C (FF/I) per ml (corr. to 5 mg of phospholipid per ml) leads to an action. This action (measured on PaO₂) is already in the order of magnitude of a 2 mg/kg rSP-C (FF/I) dose (corr. to 100 mg/kg of phospholipid). The use of a rinsing solution of 20 µg of rSP-C (FF/I) per ml (1 mg of phospholipids per ml) followed by the administration of 2 mg/kg of rSP-C (FF/I) (corr. to 100 mg/kg of phospholipid) leads to an improvement in the PaO₂ with a reduction of the standard deviation (SD).

On the one hand, these investigations show that with low concentrations of rSP-C (FF/I) surfactant [100 µg of rSP-C (FF/I) per ml (corr. to 5 mg of phospholipid per ml)], effects are achievable which can be obtained with higher doses after instillation (concentrations of 0.5 mg/ml of rSP-C (FF/I), corresponding to 25 mg/ml of phospholipid). On the other hand, it is seen that an amount as low as 400 µg/kg of rSP-C (FF/I) (20 mg/kg of phospholipid) suffices in order to obtain an adequate action. This means a lower stress on the lung with surfactant. In the case of rinsing and subsequent instillation, it can mean the action of the instilled surfactant lasts longer and the surfactant is better dispersed in the lung after rinsing. In addition, the group which was lavaged with NaCl solution only lead to no improvements with regard to oxygenation. Combining NaCl solution rinsing with instillation of surfactant lead also not to improvements in oxygenation when compared to intratracheal instillation alone. This finding supports the idea that only rinsing with a surfactant solution above a certain surfactant concentration followed by intratracheal surfactant instillation lead to improvements.

### Description of the drawing

### Figure 1:

Influence of intratracheal administration and rinsing with NaCl solution or a surfactant solution and the combination of both in the rat lung lavage model. The partial arterial oxygen pressure (PaO₂ [mm Hg]; indicated as a mean value ± SD) is indicated at the experimental time 180 min after the last lavage. The administration of surfactant and rinsing with surfactant was carried out 60 min after the last lavage. The rinsing solution was always instilled at a volume of 8 ml/animal in the concentrations indicated, or using NaCl solution (line 1 of the abscissa, conc. of LSF solution). In line 2 of the abscissa, the amount of surfactant administered (intratrach. administration in mg of phospholipid per kg of body weight) is shown. Line 3 represents the amount of surfactant administered in mg per animal. The last line indicates the amount of surfactant remaining in the animal (estimation) also in mg per animal.

## Claims

1. A set for the treatment of IRDS or ARDS, comprising a first container which has a volume of 50 to 500 ml and contains a pulverulent pulmonary surfactant preparation, the amount of phospholipids in the surfactant being 50 to 500 mg, and a second container which has a volume of 50 to 500 ml and contains a pulverulent pulmonary surfactant preparation, the amount of phospholipids in the surfactant of the second container being 1 to 10 g and wherein a pulmonary surfactant protein is only contained in the second container.

2. The set as claimed in claim 1, wherein there are two containers having an identical volume of 250 or 500 ml.

3. The set as claimed in claim 2, wherein the pulmonary surfactant protein is SP-C, SP-B and/or their modified derivatives.

4. The set as claimed in claim 3, wherein the pulmonary surfactant preparation in the second container contains 20 to 200 mg of rSP-C (FF/I).

5. The set as claimed in any of claims 1 to 4, wherein the pulmonary surfactant preparations in the first container and second container are dissolved in a solvent and wherein the pulmonary surfactant solution of the first container contains 1 to 5 mg of phospholipids per mi of solvent and wherein the pulmonary surfactant solution of the second container contains 25 to 100 mg of phospholipids per ml of solvent.

6. The set as claimed in any of claims 1 to 5, wherein the pulmonary surfactant solution in the second container contains 0.5 to 2.0 mg of rSP-C (FF/I) per ml of solvent.

7. The set as claimed in any of claims 1 to 6, wherein the solvent is a physiological saline solution.

8. Article of manufacture comprising customary secondary packaging material and a set for treatment according to claim 1 contained within the packaging material, optionally together with suitable pharmaceutical auxiliaries and/or suitable devices for administration of pulmonary surfactant and wherein a container and/or the secondary packaging material comprises a label or package insert which indicates that the pulmonary surfactant preparation contained in the container is useful for preventing or treating pneumonia, bronchitis, meconium aspiration syndrome, COPD (chronic obstructive pulmonary disease), asthma, cystic fibrosis, IRDS and/or ALI (including ARDS).

## Patentansprüche

1. Set zur Behandlung von IRDS oder ARDS, umfassend ein erstes Behältnis mit einem Volumen von 50 bis 500 ml, das eine pulverförmige Lungensurfactantzubereitung enthält, wobei die Menge an Phospholipiden in dem Surfactant 50 bis 500 mg beträgt, und ein zweites Behältnis mit einem Volumen von 50 bis 500 ml, das eine pulverförmige Lungensurfactantzubereitung enthält, wobei die Menge an Phospholipiden in dem Surfactant des zweiten Behältnisses von 1 bis 10 g beträgt und wobei nur im zweiten Behältnis ein Lungensurfactantprotein enthalten ist.

2. Set nach Anspruch 1, wobei zwei Behältnisse mit einem identischen Volumen von 250 oder 500 ml vorliegen.

3. Set nach Anspruch 2, wobei es sich bei dem Lungensurfactantprotein um SP-C, SP-B und/oder deren modifizierte Derivate handelt.

4. Set nach Anspruch 3, wobei die Lungensurfactantzubereitung im zweiten Behältnis 20 bis 200 mg rSP-C (FF/I) enthält.

5. Set nach einem der Ansprüche 1 bis 4, wobei die Lungensurfactantzubereitungen im ersten Behältnis und im zweiten Behältnis in einem Lösungsmittel gelöst sind und wobei die Lungensurfactantlösung des ersten Behältnisses 1 bis 5 mg Phospholipide pro ml Lösungsmittel enthält und wobei die Lungensurfactantlösung des zweiten Behältnisses 25 bis 100 mg Phospholipide pro ml Lösungsmittel enthält.

6. Set nach einem der Ansprüche 1 bis 5, wobei die Lungensurfactantlösung im zweiten Behältnis 0,5 bis 2,0 mg rSP-C (FF/I) pro ml Lösungsmittel enthält.

7. Set nach einem der Ansprüche 1 bis 6, wobei es sich bei dem Lösungsmittel um physiologische Kochsalzlösung handelt.

8. Handelsprodukt, umfassend ein übliches Sekundärpackmittel und ein Set zur Behandlung nach Anspruch 1 in dem Packmittel, gegebenenfalls zusammen mit geeigneten pharmazeutischen Hilfsstoffen und/oder geeigneten Vorrichtungen zur Verabreichung von Lungensurfactant, wobei ein Behältnis und/oder das Sekundärpackmittel ein Etikett oder einen Beipackzettel enthält, in dem darauf hingewiesen wird, daß sich die in dem Behältnis enthaltene Lungensurfactantzubereitung zur Prävention bzw. Behandlung von Lungenentzündung, Bronchitis, Mekoniumaspirationssyndrom, COPD (chronisch-obstruktiver Lungenerkrankungen), Asthma, zystischer Fibrose, IRDS und/oder ALI (einschließlich ARDS) eignet.

## Revendications

1. Ensemble destiné au traitement de l'IRDS ou de l'ARDS comprenant : un premier conteneur qui a un volume de 50 à 500 ml et qui contient une préparation en poudre de surfactant pulmonaire, la quantité de phospholipides dans le surfactant étant de 50 à 500 mg ; et un deuxième conteneur qui a un volume de 50 à 500 ml et qui contient une préparation en poudre de surfactant pulmonaire, la quantité de phospholipides dans le surfactant du deuxième conteneur étant de 1 à 10 g ; et dans lequel une protéine du surfactant pulmonaire est contenue seulement dans le deuxième conteneur.

2. Ensemble selon la revendication 1, dans lequel il y a deux conteneurs ayant un volume identique de 250 ou de 500 ml.

3. Ensemble selon la revendication 2, dans lequel la protéine du surfactant pulmonaire est la SP-C, la SP-B et/ou leurs dérivés modifiés.

4. Ensemble selon la revendication 3, dans lequel la préparation de surfactant pulmonaire dans le deuxième conteneur contient 20 à 200 mg de rSP-C (FF/I).

5. Ensemble selon l'une quelconque des revendications 1 à 4, dans lequel les préparations de surfactant pulmonaire dans le premier conteneur et dans le deuxième conteneur sont dissoutes dans un solvant ; dans lequel la solution de surfactant pulmonaire du premier conteneur contient 1 à 5 mg de phospholipides par ml de solvant ; et dans lequel la solution de surfactant pulmonaire du deuxième conteneur contient 25 à 100 mg de phospholipides par ml de solvant.

6. Ensemble selon l'une quelconque des revendications 1 à 5, dans lequel la solution de surfactant pulmonaire dans le deuxième conteneur contient 0,5 à 2,0 mg de rSP-C (FF/I) par ml de solvant.

7. Ensemble selon l'une quelconque des revendications 1 à 6, dans lequel le solvant est une solution saline physiologique.

8. Article manufacturé comprenant un matériau coutumier d'emballage secondaire et un ensemble destiné à un traitement, selon la revendication 1, contenu dans le matériau d'emballage, se trouvant en option conjointement avec des auxiliaires pharmaceutiques appropriés et/ou des dispositifs appropriés pour l'administration d'un surfactant pulmonaire et dans lequel un conteneur et/ou le matériau d'emballage secondaire comprennent un marqueur ou un insert dans l'emballage qui indique que la préparation de surfactant pulmonaire contenue dans le conteneur est utile pour prévenir ou traiter un pneumonie, une bronchite, le syndrome d'aspiration méconiale, une COPD (maladie pulmonaire obstructive chronique), l'asthme, une fibrose cystique, l'IRDS et/ou l'ALI (y compris l'ARDS).
